# EUROPEAN PATENT SPECIFICATION

(11) **EP 3 381 903 B1**
(45) Date of publication and mention of the grant of the patent: **30.12.2020**
(21) Application number: 16868689.7
(22) Date of filing: 25.11.2016
(51) Int. Cl.: C07D 213/71, C07D 213/73, C07D 213/74, C07D 213/76, C07D 213/84

(54) **PENTAFLUOROSULFANYLPYRIDINE**
PENTAFLUORSULFANYLPYRIDINE
PENTAFLUOROSULFANYLPYRIDINE

(30) Priority: 25.11.2015 JP 2015229882; 12.07.2016 JP 2016137763
(43) Date of publication of application: 03.10.2018
(73) Proprietor: UBE Industries, Ltd., Yamaguchi 755-8633 (JP)
(72) Inventor: SHIBATA, Norio, Nagoya-shi Aichi 466-0061 (JP); MATSUZAKI, Kohei, Nagoya-shi Aichi 466-0061 (JP); SAITO, Norimichi, Ube-shi Yamaguchi 755-8633 (JP)
(74) Representative: Mewburn Ellis LLP
(86) International application number: PCT/JP2016/085036
(87) International publication number: WO 2017/090746

(56) References cited:
- WO-A1-2011/105506
- WO-A1-2014/119674
- CN-A- 105 985 284
- JP-A- H08 508 476
- JP-A- 2002 532 477
- JP-A- 2013 136 519
- JP-A- 2014 513 037
- US-A- 5 739 326
- KANISHCHEV, OLEKSANDR S. et al.: "Synthesis and Characterization of 2-Pyridylsulfur Pentafluorides", Angew. Chem. Int. Ed., vol. 54, no. 1 2 January 2015 (2015-01-02), pages 280-284, XP055385365, ISSN: 1521-3773 Retrieved from the Internet: URL:DOI: 10.1002/anie.201409990
- MASAHIRO TAKADA et al.: "Chlorotetrafluorosulfanyl(SF4Cl)-ki o Motsu Pyridine-rui no Gosei to Hanno [Synthesis and reaction of (chlorotetrafluoro-[gamma]6-sulfanyl)pyrid ines]", Dai 46 Kai Congress of Heterocyclic Chemistry Koen Yoshishu, 9 September 2016 (2016-09-09), pages 257-258, XP009511369,
- KOSOBOKOV, MIKHAIL et al.: "Importance of a Fluorine Substituent for the Preparation of meta- and para-Pentafluoro- lambda6-sulfanyl- Substituted Pyridines", Angew. Chem. Int. Ed., vol. 55, no. 36 26 August 2016 (2016-08-26), pages 10781-10785, XP055490491, ISSN: 1521-3773 Retrieved from the Internet: URL:https://doi.org/10.1002/anie.201605008
- MASAHIRO TAKADA et al.: "Chlorotetrafluorosulfanyl-ki o Yusuru Pyridine Kagobutsu no Gosei Kenkyu", Dai 39 Kai Japanese Symposium on Fluorine Chemistry Koen Yoshishu, 29 September 2016 (2016-09-29), pages 152-153, XP009511620, ISSN: 1880-6201

## Description

### [Technical Field]

The present invention relates to pentafluorosulfanyl pyridine and a manufacturing process of the same.

### [Background Art]

The pentafluorosulfanyl group (hereinafter referred to as the SF₅ group) is an electron-withdrawing and lipophilic substituent used for designing a functional organic molecule. Accordingly, a SF₅ group-containing compound is crucial in the development of a liquid crystal molecule or a low molecular weight medicine/agricultural chemical molecule, and the synthetic method of such compound is recently under development (NPL1). It is particularly important to introduce a SF₅ group to the pyridine ring, which is widely used as biologically active substances in pharmaceutical and agrochemicals, but the introduction of SF5 group is difficult, due to the instability of the intermediates, namely trifluorosulfanyl pyridine and halotetrafluorosulfanyl pyridine. Proposed as a method for introducing SF₅ group to pyridine ring is the one using a disulfide compound as a starting material, and the disulfide is oxidized in the presence of potassium fluoride by chlorine gas and then the subjecting obtained material, through a halogen exchange reaction using silver fluoride, is converted to the compound having a SF₅ group at position-2 of the pyridine ring (NPL2). However, a pragmatic method for introducing a SF₅ group to position-3 or position-4 of the pyridine ring has yet to be developed. Although there is report that a SF₅ group may be introduced to position-3 of the pyridine ring by fluorination of pyridyldisulfide using AgF₂ (PTL1), only a single sentence is attributed to the fact, and no data of the compound such as NMR or Examples of the compound are shown. There are also other reports of bioactive compound groups that have a SF₅ group introduced onto position-3 of the pyridine ring (PTL2 to PTL5), but similarly to the above, only a single sentence is attributed to the fact, and no data of the compound or Examples of the compound are shown. JP 2013-136519 A concerns a fused heterocyclic compound. JP 2014-513037 A concerns industrial methods for producing arylsuofur pentafluorides.

There is no report of a compound having a SF₅ group on position-4 of a pyridine ring, and of a compound having a chlorotetrafluorosulfanyl group (hereinafter referred to as the SF₄Cl group) on position-3 or position-4 of the pyridine ring.

### [Citation List]

### [Patent Literature]

[PTL1] WO 1994/022817
[PTL2] US Patent No. 5739326
[PTL3] US Patent No. 6531501
[PTL4] WO 2011/105506
[PTL5] WO 2014/119674

### [Non Patent Literature]

[NPL1]Chem. Rev. 2015, 115, 1130.
[NPL2]Angew. Chem. Int. Ed. 2015, 54, 280.

### [Summary of Invention]

### [Technical Problem]

The synthesis of SF₅-containing pyridine is extremely difficult because the intermediates are unstable, and the only case reported to have been successfully synthesized so far is a compound having a SF₅ group on position-2 of the pyridine ring. On the other hand, if a compound having a SF₅ group or its precursor, a SF₄Cl group, bound to a position other than position-2 of the pyridine ring is successfully synthesized, such compound would greatly benefit the design of the next generation medicine/agricultural chemical molecule. In view of this circumstance, the present invention is directed to the issue of providing a pyridine having a SF₅ group or a SF₄Cl group on position-3 or position-4 of the pyridine ring and a manufacturing process of the same.

### [Solution of Problem]

The present inventors found that the above issue can be solved by using pyridyldisulfide that possesses specific substituents as a starting material, and completed the present invention. In other words, the aforementioned issue may be solved by the present invention described below.
(1) Pentafluorosulfanyl pyridine represented by formula (d) shown below, wherein a SF₅ group is substituted on either position-3 or position-4 of a pyridine ring, and wherein at least one of the R¹ and R⁴ is a fluorine atom.
(2) Pentafluorosulfanyl pyridine according to (1) that is 2-fluoro-3-pentafluorosulfanyl pyridine, 2,6-difluoro-3-pentafluorosulfanyl pyridine, or 6-fluoro-3-pentafluorosulfanyl pyridine.
(3) Chlorotetrafluorosulfanyl pyridine represented by formula (c') shown below, wherein a SF₄Cl group is substituted on either position-3 or position-4 of a pyridine ring, and wherein at least one of the R¹ and R⁴ is a fluorine atom.
(4) Chlorotetrafluorosulfanyl pyridine according to (3) that is 2-fluoro-3-chlorotetrafluorosulfanyl pyridine, 2,6-difluoro-3-chlorotetrafluorosulfanyl pyridine, or 6-fluoro-3-chlorotetrafluorosulfanyl pyridine.
(5) A manufacturing process of pentafluorosulfanyl pyridine according to (1) comprising: a step of reacting pyridyldisulfide represented by formula (a) shown below with a halogen selected from a group consisting of chlorine, bromine and iodine, or an interhalogen compound selected from a group consisting of ClF, BrF, ClBr, ClI, Cl₃I and BrI, and a fluoride salt represented by formula (b) shown below to form halotetrafluorosulfanyl pyridine represented by formula (c) shown below; and a step of reacting the obtained halotetrafluorosulfanyl pyridine with a fluoride source to form pentafluorosulfanyl pyridine.
(6) The process according to (5), wherein the halogen to be reacted with the pyridyldisulfide compound is chlorine (Cl₂).
(7) The process according to (5), wherein the fluoride salt represented by formula (b) is alkali metal fluoride.
(8) The process according to any one of (5) to (7), wherein the fluoride source is hydrogen fluoride.

### [Advantageous Effect of Invention]

The present invention may provide manufacturing of a pentafluorosulfanyl pyridine in which position-3 or position-4 of the pyridine ring is substituted with a SF₅ group and a manufacturing process of this compound.

### [Description of Embodiments]

The present invention is described in detail below. In this invention, the expression "X to Y" includes the values on both ends, that is, X and Y.

### 1. Pentafluorosulfanyl pyridine

The pentafluorosulfanyl pyridine of the present invention is represented by formula (d). 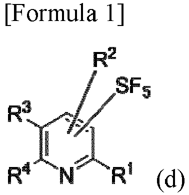 wherein the SF₅ group binds to the pyridine ring at position-3 or position- 4. R² binds to one of position-3 and position-4 that the SFs group does not bind to.

R¹, R², R³, and R⁴ are substituents on the pyridine ring, which are independently a hydrogen atom, a halogen atom, an alkyl group having 1 to 18 carbon atoms, an aryl group having 6 to 30 carbon atoms, a nitro group, a cyano group, an alkanesulfonyl group having 1 to 18 carbon atoms, an arenesulfonyl group having 6 to 30 carbon atoms, an alkoxy group having 1 to 18 carbon atoms, an aryloxy group having 6 to 30 carbon atoms, an acyloxy group having 1 to 18 carbon atoms, an alkanesulfonyloxy group having 1 to 18 carbon atoms, an arenesulfonyloxy group having 6 to 30 carbon atoms, an alkoxycarbonyl group having 2 to 18 carbon atoms, an aryloxycarbonyl group having 7 to 30 carbon atoms, a benzylamino group, an amino group, an azide group, an aralkyloxy group having 7 to 30 carbon atoms, an aralkylsulfide group having 7 to 30 carbon atoms, or a SFs group. The halogen atom is a fluorine atom, a chlorine atom, a bromine atom, or an iodine atom. An aralkyloxy group having 7 to 30 carbon atoms includes a benzyloxy group (Ph-CH₂-O-). An aralkylsulfide group having 7 to 30 carbon atoms includes a benzylsulfide group (Ph-CH₂-S-). At least one of the R¹ and R⁴ is a fluorine atom.

Due to the availability of the halotetrafluorosulfanyl compound, which is a precursor of Compound (d), R¹ and R² should preferably be a hydrogen atom or a fluorine atom, which exhibit low steric hindrance.

In view of the stability of the halotetrafluorosulfanyl compound, which is a precursor of Compound (d), at least one of R¹ and R⁴ should be a fluorine atom.

In addition, at least one of R¹ and R⁴ should preferably be a benzylamino group, an amino group, an azide group, an aralkyloxy group having 7 to 30 carbon atoms, or an aralkylsulfide group having 7 to 30 carbon atoms.

### 2. Chlorotetrafluorosulfanyl pyridine

The chlorotetrafluorosulfanyl pyridine of the present invention is represented by formula (c'). 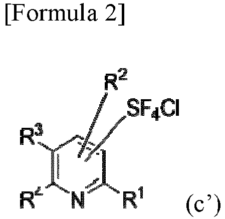 wherein SF₄Cl group is a group that provides the SF₅ group to Compound (d). R¹, R², R³, and R⁴ are defined in the same manner as Compound (d).

### 3. Manufacturing process of pentafluorosulfanyl pyridine

It is preferable for pentafluorosulfanyl pyridine to be manufactured by the following scheme.

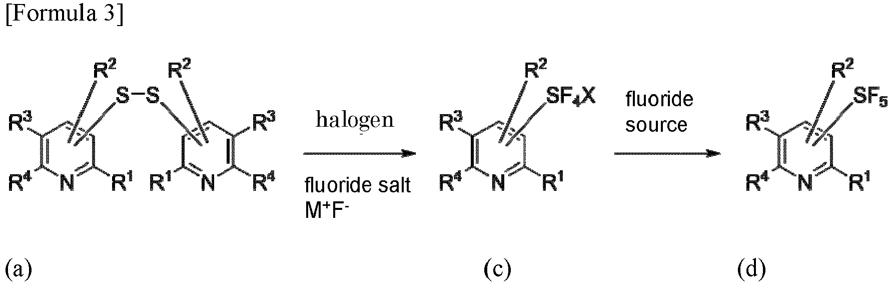

### (1) Synthesis of halotetrafluorosulfanyl pyridine (c)

The pyridyldisulfide compound represented by formula (a) is reacted with a halogen selected from a group consisting of chlorine, bromine, iodine and interhalogen compounds, and a fluoride salt represented by formula (b) to manufacture halotetrafluorosulfanyl pyridine compound represented by formula (c).

Specifically speaking, the pyridyldisulfide compound (Compound (a)) is first reacted with a fluoride salt under the presence of a halogen to obtain halotetrafluorosulfanyl pyridine (Compound (c)). The amount of halogen used may be any amount as long as it is excessive against the pyridyldisulfide compound, but an amount of 12 to 16 equivalents is preferred. The amount of fluoride salt to be used may be any amount as long as it is excessive against pyridyldisulfide, but an amount of 14 to 18 equivalents is preferred. There is no limitation to the solvent, but an aprotic, polar solvent, such as acetonitrile, is preferred in view of the solubility of the fluoride salt and to prevent the fluoride salt's reaction with halogens. The reaction temperature may be adjusted as necessary, but a temperature of -20 to 50°C is preferred. The reaction time is not limited, but it may be from a few hours to a few days, preferably 2 to 3 days. The reaction container is preferably that made from fluoropolymer, such as polytetrafluoroethylene, in order to suppress secondary reactions. In addition, the reaction should preferably be performed under a deoxidized atmosphere and a dry condition in view of the instability of the reaction intermediates.

The R¹, R², R³, and R⁴ in Compound (d) may differ from R¹, R², R³, and R⁴ of the starting material, Compound (a). Accordingly, some embodiments of the present invention includes transforming R¹, R², R³, and R⁴ to a different R¹, R², R³, and R⁴. This transformation may be performed in a reaction that converts Compound (a) to Compound (c) or in a reaction that transforms Compound (c) to Compound (d).

Typical halogens to be used in the present invention include chlorine (Cl₂), bromine (Br₂), iodine (I₂), and interhalogen compounds such as ClF, BrF, ClBr, ClI, Cl₃I, BrI. Chlorine (Cl₂) is preferable from the cost perspective.

The fluoride salt represented by formula (b) is easily acquired, and examples of such fluoride salt include metal fluorides, ammonium fluorides and phosphonium fluorides. Examples of preferable metal fluorides are alkali metal fluorides, such as lithium fluoride, sodium fluoride, potassium fluoride (including spray-dried potassium fluoride), rubidium fluoride, and caesium fluoride. Examples of preferable ammonium fluorides are tetramethyl ammonium fluoride, tetrabutyl ammonium fluoride, benzyltrimethyl ammonium fluoride, and benzyltriethyl ammonium fluoride. Examples of preferable phosphonium fluorides are tetramethyl phosphonium fluoride, tetraethyl phosphonium fluoride, tetrapropyl phosphonium fluoride, tetrabutyl phosphonium fluoride, tetraphenyl phosphonium fluoride, and tetratolylphosphonium fluoride. An alkali metal fluoride such as potassium fluoride and caesium fluoride are preferred from the perspective of ease of acquisition and of capacity to provide high yield, and potassium fluoride is preferred from the cost perspective.

A mixture of metal fluoride and ammonium fluoride or phosphonium fluoride, a mixture of ammonium fluoride and phosphonium fluoride, and a mixture of metal fluoride, ammonium fluoride and phosphonium fluoride may be used as a fluoride salt represented by formula (b).

### (2) Synthesis of pentafluorosulfanyl pyridine (d)

Halotetrafluorosulfanyl pyridine represented by formula (c) is reacted with a fluoride source to obtain pentafluorosulfanyl represented by formula (d).

Specifically speaking, halotetrafluorosulfanyl pyridine (Compound (c)) is reacted with a fluoride source to obtain pentafluorosulfanyl pyridine (Compound (d)) by a halogen exchange reaction. The amount of fluoride source used may be any amount as long as it is excessive against pyridyldisulfide, but an amount of 12 to 16 equivalents is preferred. The amount of fluoride source to be used may be any amount as long as it is excessive against halotetrafluorosulfanyl pyridine, but an amount of 1 to 3 equivalents is preferred. A solvent-less reaction is preferred from the perspective of high reaction efficiency and low cost, but a halogen-containing carbon compound, ether, nitrile (acetonitrile, etc.), or nitro compound may also be used as a solvent to adjust reactivity. The reaction temperature may be adjusted as necessary, but a temperature of -100 to 150°C is preferred. The reaction time is not limited, but it may be from a few hours to a few days, preferably 2 to 3 days. The reaction container is preferably that made from fluoropolymer, such as polytetrafluoroethylene, in order to suppress secondary reactions. In addition, the reaction should preferably be performed under a deoxidized atmosphere and a dry condition from the perspective of instability of reaction intermediates.

The fluoride source to be used is an anhydride compound exhibiting an activity of fluorinating halotetrafluorosulfanyl pyridine represented by formula (c). The fluoride source may be selected from a fluoride of a typical element in the periodic table, a fluoride or a transition element in the periodic table, and a mixture or a compound of fluorides of the typical element or the transition element. The fluoride source may be hydrogen fluoride or a mixture, salt or complex with a single or multiple types of organic molecules that does not limit the present invention. The fluoride source also includes a mixture or a compound of the fluoride source and a fluoride source-activating compound, such as SbCl₅, AlCl₃, PCl₅, BCl₃. Fluorides of the Group 11 elements (Cu, Ag, Au) and Group 12 elements (Zn, Cd, Hg), which are transition metal fluorides, are preferred from the perspective of high reactivity of halogen exchange.

The present process is categorized into reactions performed in stages as shown below. 1) Oxidation of the sulfur atom binding to position-3 or position-4 of the pyridine ring by halogen and fluoride salt, and generation of halotetrafluorosulfanyl compound by halogen exchange. 2) Generation of pentafluorosulfanyl pyridine compound by halogen exchange.

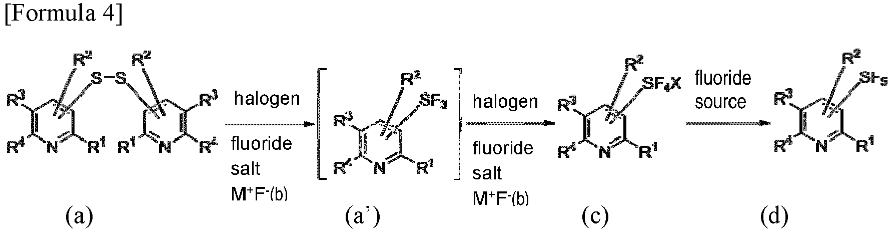

In Stage 1) above, a compound including a SF₃ group represented by formula (a') is provided as an intermediate. The compound is a tetravalent sulfur compound, and its presence may be verified by ¹⁹F-NMR A pyridine compound possessing a SF₃ group or a SF₄Cl group is easily decomposed by a nucleophilic attack by water or an anion type in the system. As such, R¹ or R⁴ that is a fluorine atom improves the chemical stability of pyridyldisulfide such that this reaction may be carried out more efficiently.

By reacting pentafluorosulfanyl pyridine (d), whose R¹ or R⁴ is a fluorine atom, with a nucleophilic reagent, it is possible to substitute R¹ or R⁴ with another functional group. The scheme of the present invention and an example of the thus synthesized pentafluorosulfanyl pyridine (d) is shown below.

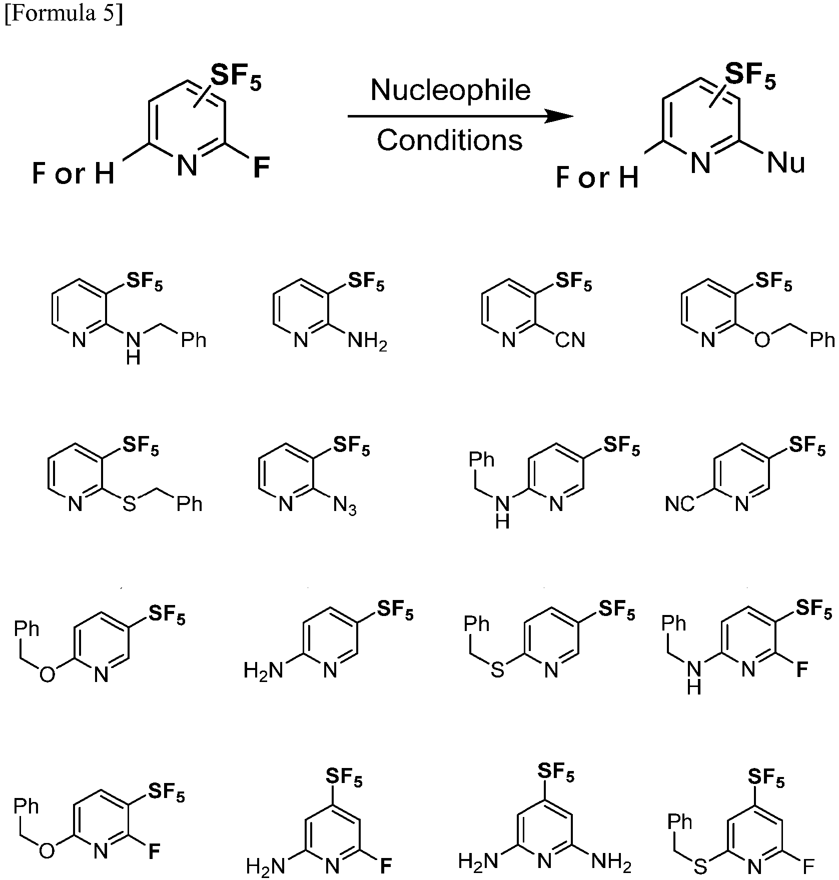

### EXAMPLES

### [Example 1]

The following reaction was performed to synthesize a pentafluorosulfanyl pyridine compound.

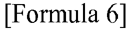

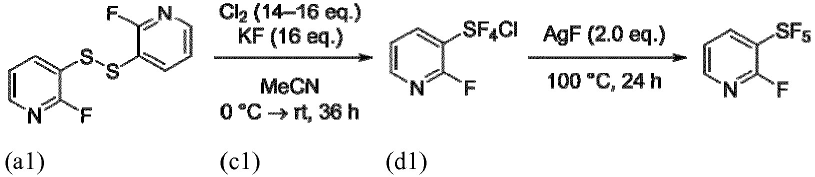

Potassium fluoride (spray-dried product, 2.32 g, 40 mmol, Wako pure chemical corp.) and acetonitrile (15 mL) were put in a 30 mL polytetrafluoroethylene container under a nitrogen atmosphere. The agitated mixture was cooled in an ice-containing water bath, and a polytetrafluoroethylene tube (inner diameter 1 mm) was used to perform bubbling of chlorine (Cl₂; about 2.5 g to 2.8 g, about 35 to 40 mmol) in the mixture for 10 min. at an outlet pressure of 0.005 MPa. Then, pyridyldisulfide (a1) (641 mg, 2.5 mmol) synthesized by the inventors was added under a nitrogen gas flow, and the container was sealed to perform agitation at room temperature for 36 h. After the reaction completed, the insoluble matter was removed under a nitrogen atmosphere using a glass filter. The filtrate was evaporated in a polytetrafluoroethylene container under vacuum, and the obtained residue (2-fluoro-3-chlorotetrafluorosulfanyl pyridine (c1)) was used for the subsequent reaction without being purified.

To the 2-fluoro-3-chlorotetrafluorosulfanyl pyridine (c1) in the 30 mL polytetrafluoroethylene container, obtained in the previous step, was added silver fluoride (I) (634 mg, 5.0 mmol), and the mixture was agitated under a nitrogen atmosphere at 100°C for 24 h. After cooling to room temperature, water (5 mL) and dichloromethane (5 mL) were added to the mixture, and the mixture was agitated for 1 h., then, the product was extracted using dichloromethane and the organic phase was dried by sodium sulfate. Then, the solvent was removed by distillation under reduced pressure, and purified by silica gel column chromatography (pentane/dichloromethane = 5/1→2/1, Rf value 0.56) and a transparent, oil-like product (d1) (151 mg, 27%) was obtained.

Similar syntheses were performed using the following compounds. The analysis result using mass spectrometry and NMR is summarized below. Mass spectrometry was performed in the present invention using a device by Shimadzu corporation with a serial name of LCMS-2020, and ¹H-NMR and ¹⁹F-NMR were performed using Mercury 300 by Varian.

### [Table 1]

**Table 1 EXAMPLE 1**

| Pyridyldisulfide | Chlorotetrafluorosulfanyl Pyridine | Pentafluorosulfanyl Pyridine |
|---|---|---|
| 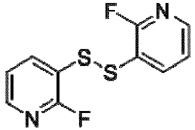 | 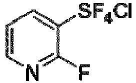 | 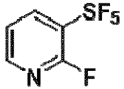 |
| a1 | c1 | d1 |
| | | 24% yield |
| 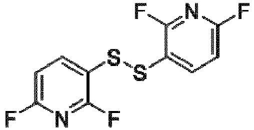 | 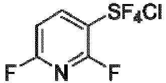 | 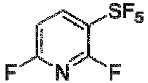 |
| a2 | c2 | d2 |
| | | 27% yield |
| 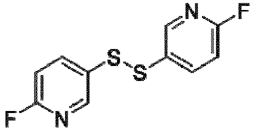 | 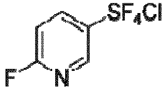 | 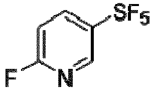 |
| a3 | c3 | d3 |
| | | 20% yield |

### [Formula 7]

### 3-(Chlorotetrafluorosulfanyl)-2-fluoropyridine (c1)

¹H NMR (CDCl₃, 300 MHz): δ = 7.36 (dd, *J* = 6.5, 5.5 Hz, 1H), 8.20 (td, *J* = 8.3, 1.7 Hz, 1H), 8.40 (d, *J* = 4.5 Hz, 1H);
¹⁹F NMR (CDCl₃, 300 MHz): δ = -59.56 - -60.48 (m, 1F) 138.71 (d, *J* = 21.5 Hz, 4F)

### 3-(Chlorotetrafluorosulfanyl)-2,6-difluoropyridine (c2)

¹⁹F NMR (CDCl₃, 300 MHz): δ = -61.86 (s, 1F), -60.32 - -61.06 (m, 1F), 139.13 (d, *J* = 21.8 Hz, 4F)

### 5-(Chlorotetrafluorosulfanyl)-2-fluoropyridine (c3)

¹⁹F NMR (CDCl₃, 300 MHz): δ = -63.88 (s, 1F), 136.28 (s, 4F)

### 2-Fluoro-3-(pentafluorosulfanyl)pyridine (d1)

MS (EI, *m*/*z*) 223 (M⁺); ¹H NMR (CDCl₃, 300 MHz): δ = 7.42 - 7.30 (m, 1H), 8.26 - 8.13 (m, 1H), 8.40 (d, *J* = 3.2 Hz, 1H); ¹⁹F NMR (CDCl₃, 300 MHz): δ = -60.21 - -61.04 (m, 1F), 66.93 (dd, *J* = 151.8, 24.0 Hz, 4F), 80.60 - 77.84 (m, IF); (2 steps) 24% yield

### 2,6-Difluoro-3-(pentafluorosulfanyl)pyridine (d2)

MS (EI, *m*/*z*) 241 (M⁺); ¹H NMR (CDCl₃, 300 MHz): δ =2 6.99 (d, *J* = 8.6 Hz, 1H), 8.51 - 8.06 (m, 1H); ¹⁹F NMR (CDCl₃, 300 MHz): δ = -63.13 (s, 1F), -61.48 - -62.07 (m, 1F), 64.79 (ddd, *J* = 150.9, 24.1, 3.6, 4F), 77.26 - 74.64 (m, 1F); (2 steps) 27% yield

### 2-Fluoro-5-(pentafluorosulfanyl)pyridine (d3)

MS (EI, *m*/*z*) 223 (M⁺); ¹HNMR (CDCl₃, 300 MHz): δ = 7.07 (d, *J* = 8.9 Hz, 1H), 8.19 (ddt, *J* = 9.1, 6.5, 2.5 Hz, 1H), 8.70 - 8.62 (br, 1H); ¹⁹F NMR (CDCl₃, 300 MHz): δ = -61.77 (s, 1F), 65.75 (d, *J* = 151.4 Hz, 4F), 82.97 - 80.04 (m, 1F); (2 steps) 20% yield

### [Example 2]

A pentafluorosulfanyl pyridine compound was synthesized by the following reaction.

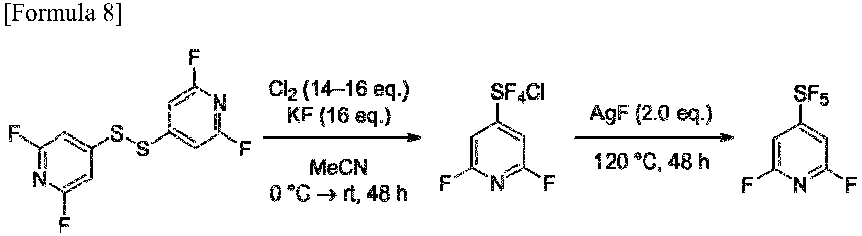

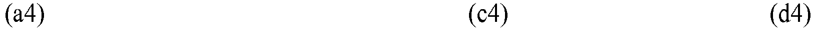

Potassium fluoride (spray-dried product, 6.03 g, 104 mmol, Wako pure chemical corp.) and acetonitrile (39 mL) were put under a nitrogen atmosphere in a 60 mL polytetrafluoroethylene container. The agitated mixture was cooled in an ice-containing water bath, and a polytetrafluoroethylene tube (inner diameter 1 mm) was used to bubble chlorine (Cl₂; about 6.5 g to 7.4 g, about 91 to 104 mmol) in the mixture for 10 min. at an outlet pressure of 0.005 MPa. Then, pyridyldisulfide (a4) (1.90 g, 6.5 mmol) synthesized by the inventors was added under a nitrogen gas flow, and the container was sealed to perform agitation at room temperature for 48 h. After the reaction completed, the insoluble matter was removed under a nitrogen atmosphere using a glass filter. The filtrate was evaporated in a polytetrafluoroethylene container under vacuum, and the obtained residue (2,6-difluoro-4-chlorotetrafluorosulfanyl pyridine (c4), 3.01 g, 90% yield) was used for the subsequent reaction without being purified.

To the previously obtained 2,6-difluoro-4-chlorotetrafluorosulfanyl pyridine (c4) (2.58 g, 10 mmol) in the 30 mL polytetrafluoroethylene container was added silver fluoride (I) (2.54 g, 20 mmol), and the mixture was agitated under a nitrogen atmosphere at 120°C for 48 h. After cooling to room temperature, water (10 mL) and dichloromethane (10 mL) were added to the mixture, and the mixture was agitated for 1 h., then, the product was extracted using dichloromethane and the organic phase was dried by sodium sulfate. Then, the solvent was removed by distillation under reduced pressure, and purified by silica gel column chromatography (pentane/dichloromethane=20/1, Rf value 0.34) and a transparent, oil-like product (d4) (844 mg, 35% yield) was obtained.

A similar synthesis was further performed and product (d5) was obtained. The analysis result using mass spectrometry and NMR is summarized below.

### [Table 2]

**Table 2 Example 2**

| Pyridyldisulfide | Chlorotetrafluorosulfanyl Pyridine | Pentafluorosulfanyl Pyridine |
|---|---|---|
| 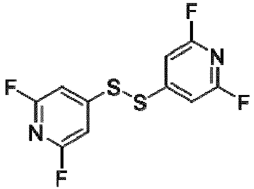 | 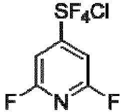 | 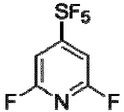 |
| a4 | c4 | d4 |
| | 90% yield | 35% yield |
| 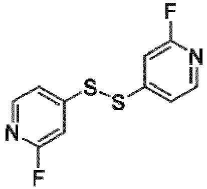 | 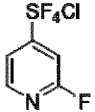 | 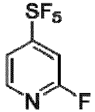 |
| a5 | c5 | d5 |
| | 68% yield | 5% yield |

### [Formula 9]

### 4-(Chlorotetrafluorosulfanyl)-2,6-difluoropyridine (c4)

¹H NMR (300 MHz, CDCl₃) δ: 7.20 (s, 2H). ¹⁹F NMR (282 MHz, CDCl₃) δ: 132.28 (s, 4F), - 63.35 (s, 2F); 90% yield

### 4-(Chlorotetrafluorosulfanyl)-2-fluoropyridine (c5)

¹H NMR (300 MHz, CDCl₃) δ: 8.42 (d, *J* = 4.7 Hz, 1H), 7.53 (d, *J* = 4.7 Hz, 1H), 7.29 (s, 1H). ¹⁹F NMR (282 MHz, CDCl₃) δ: 132.48 (s, 4F), -63.54 (s, 1F); 68% yield

### 2,6-Difluoro-4-(pentafluorosulfanyl)pyridine (d4)

HRMS (TOF/EI+): Calculated for C₅H₂F₇NS⁺: 240.9796, found: 240.9799; ¹H NMR (300 MHz, CDCl₃) δ: 7.22 (s, 2H). ¹⁹F NMR (282 MHz, CDCl₃) δ: 78.69 - 75.66 (m, 1F), 60.91 (d, *J* = 152.4 Hz, 4F), -63.14 (s, 2F). ¹³C NMR (125 MHz, CDCl₃) δ: 167.1 - 166.4 (m), 161.7 (dd, *J* = 250.8, 15.3 Hz), 105.0 - 104.4 (m); 35% yield

### 2-Fluoro-4-(pentafluorosulfanyl)pyridine (d5)

HRMS (TOF/EI+): Calculated for C₅H₃F₆NS⁺: 222.9890, found: 222.9888;
¹H NMR (300 MHz, CDCl₃) δ: 8.44 (d, *J* = 5.4 Hz, 1H), 7.56 (d, *J* = 5.4 Hz, 1H), 7.37 - 7.24 (m, 1H). ¹⁹F NMR (282 MHz, CDCl₃) δ: 79.85 - 76.24 (m, 1F), 60.52 (d, *J* = 151.7 Hz, 4F), - 63.43 (s, 1F). ¹³C NMR (125 MHz, CDCl₃) δ: 163.8 (pd, *J* = 20.8, 7.5 Hz), 163.8 (d, *J* = 242.6 Hz), 149.2 (d, *J* = 15.0 Hz), 118.2 - 118.0 (m), 107.8 (dp, *J* = 42.4, 4.9 Hz); 5% yield

### [Example 3]

Pentafluorosulfanyl pyridine (d1) synthesized as described above was subjected to a nucleophilic substitution reaction. Table 3 shows the nucleophilic reagent, the reaction condition and the product.

### [Table 3]

**Table 3 Example 3: Nucleophilic substitution reaction of Compound (d1)**

| Nucleophilic Reagent | Reaction Condition | Product (number shows yield) |
|---|---|---|
| benzylamine | 95°C, 10 h. | 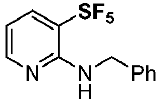 |
| | | 86% |
| NH3 | 105°C, 18 h. | 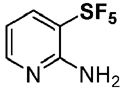 |
| | | 72% |
| MesSiCN | 55°C, 10 h. | 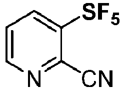 |
| | | 87% |
| benzylalcohol | 95°C, 10 h. | 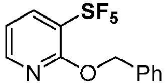 |
| | | 84% |
| benzylthiol | 95°C, 10 h. | 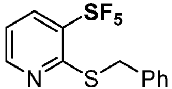 |
| | | 83% |
| NaN₃ | 95°C, 10 h. | 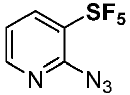 |
| | | 96% |

### [Example 4]

Pentafluorosulfanyl pyridine (d3) synthesized as described above was subjected to a nucleophilic substitution reaction. Table 4 shows the nucleophilic reagent, the reaction condition, and the product, etc.

### [Table 4]

**Table 4 Example 4: Nucleophilic substitution reaction of Compound (d3)**

| Nucleophilic Reagent | Reaction Condition | Product (number shows yield) |
|---|---|---|
| benzylamine | 95°C, 10 h. | 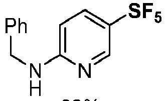 |
| | | 92% |
| Me₃SiCN | 55°C, 10 h. | 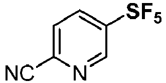 |
| | | 83% |
| benzylalcohol | 95°C, 10 h. | 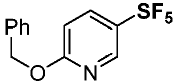 |
| | | 93% |
| NH3 | 105°C, 18 h. | 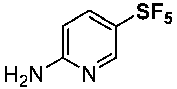 |
| | | 89% |
| benzylthiol | 95°C, 10 h. | 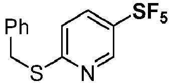 |
| | | 84% |

### [Example 5]

Pentafluorosulfanyl pyridine (d2) synthesized as described above was subjected to a nucleophilic substitution reaction. Table 5 shows the nucleophilic reagent, the reaction condition, and the product, etc.

### [Table 5]

**Table 5 Example 5: Nucleophilic substitution reaction of Compound (d2)**

| Nucleophilic Reagent | Reaction Condition | Product (number shows yield) |
|---|---|---|
| benzylamine | 25°C, 10 h. | 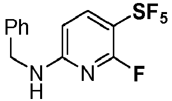 |
| | | 89% |
| benzylalcohol | 25°C, 10 h. | 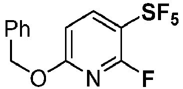 |
| | | 72% |

### [Example 6]

Pentafluorosulfanyl pyridine (d4) synthesized as described above was subjected to a nucleophilic substitution reaction. Table 6 shows the nucleophilic reagent, the reaction condition, and the product, etc.

### [Table 6]

**Table 6 Example 6: Nucleophilic substitution reaction of Compound (d4)**

| Nucleophilic Reagent | Reaction Condition | Product (number shows yield) |
|---|---|---|
| NH3 | 90°C, 3 h. | 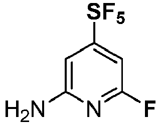 |
| | | 69% |
| NH₃ | 120°C, 24 h. | 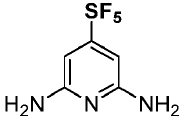 |
| | | 77% |
| benzylthiol | 95°C, 10 h. | 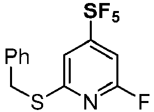 |
| | | 70% |

It is apparent that a pyridine compound having a pentafluorosulfanyl group at position-3 and position-4 are obtained by the present invention. A pyridine compound having a halogen atom together with pentafluorosulfanyl group may be synthesized in the present invention, and modification such as a coupling reaction, performed using the halogen atom, allows the invention to be of assistance in the synthesis of physiological substances such as medicine.

## Claims

1. Pentafluorosulfanyl pyridine represented by formula (d): 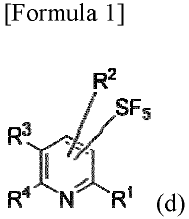
wherein a SFs group binds to either one of position-3 or position-4 of a pyridine ring, and R² binds to the other one of position-3 or position-4; and
R¹, R², R³, and R⁴ are independently a hydrogen atom, a halogen atom, an alkyl group having 1 to 18 carbon atoms, an aryl group having 6 to 30 carbon atoms, a nitro group, a cyano group, an alkanesulfonyl group having 1 to 18 carbon atoms, an arenesulfonyl group having 6 to 30 carbon atoms, an alkoxy group having 1 to 18 carbon atoms, an aryloxy group having 6 to 30 carbon atoms, an acyloxy group having 1 to 18 carbon atoms, an alkanesulfonyloxy group having 1 to 18 carbon atoms, an arenesulfonyloxy group having 6 to 30 carbon atoms, an alkoxycarbonyl group having 2 to 18 carbon atoms, an aryloxycarbonyl group having 7 to 30 carbon atoms, a benzylamino group, an amino group, an azide group, an aralkyloxy group having 7 to 30 carbon atoms, an aralkylsulfide group having 7 to 30 carbon atoms, or a SF₅ group);
wherein at least one of the R¹ and R⁴ is a fluorine atom.

2. Pentafluorosulfanyl pyridine according to claim 1 that is 2-fluoro-3-pentafluorosulfanyl pyridine, 2,6-difluoro-3-pentafluorosulfanyl pyridine, or 6-fluoro-3-pentafluorosulfanyl pyridine.

3. Chlorotetrafluorosulfanyl pyridine represented by formula (c'): 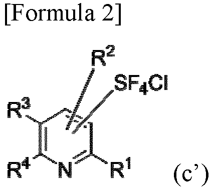
wherein a SF₄Cl group binds to either one of position-3 or position-4 of a pyridine ring, and R² binds to the other one of position-3 or position-4; and
R¹, R², R³, and R⁴ are independently a hydrogen atom, a halogen atom, an alkyl group having 1 to 18 carbon atoms, an aryl group having 6 to 30 carbon atoms, a nitro group, a cyano group, an alkanesulfonyl group having 1 to 18 carbon atoms, an arenesulfonyl group having 6 to 30 carbon atoms, an alkoxy group having 1 to 18 carbon atoms, an aryloxy group having 6 to 30 carbon atoms, an acyloxy group having 1 to 18 carbon atoms, an alkanesulfonyloxy group having 1 to 18 carbon atoms, an arenesulfonyloxy group having 6 to 30 carbon atoms, an alkoxycarbonyl group having 2 to 18 carbon atoms, an aryloxycarbonyl group having 7 to 30 carbon atoms, a benzylamino group, an amino group, an azide group, an aralkyloxy group having 7 to 30 carbon atoms, an aralkylsulfide group having 7 to 30 carbon atoms, or a SF₅ group);
wherein at least one of the R¹ and R⁴ is a fluorine atom.

4. Chlorotetrafluorosulfanyl pyridine according to claim 3 that is 2-fluoro-3-chlorotetrafluorosulfanyl pyridine, 2,6-difluoro-3-chlorotetrafluorosulfanyl pyridine, or 6-fluoro-3-chlorotetrafluorosulfanyl pyridine.

5. A manufacturing process of pentafluorosulfanyl pyridine according to claim 1 comprising:
a step of reacting a pyridyldisulfide compound represented by formula (a): 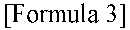 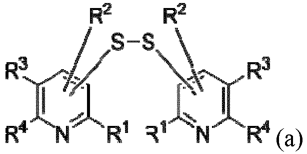
wherein R¹, R², R³, and R⁴ are as defined above,
with a halogen selected from a group consisting of chlorine, bromine and iodine, or an interhalogen compound selected from a group consisting of ClF, BrF, ClBr, ClI, Cl₃I and BrI, and
a fluoride salt represented by formula (b):
M⁺F⁻ (b)
wherein M is a metal atom, an ammonium group or a phosphonium group
to form halotetrafluorosulfanyl pyridine represented by formula (c): 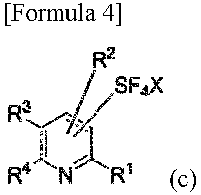
wherein R¹, R², R³, and R⁴ are as defined above, and X is a chlorine atom, a bromine atom or an iodine atom; and
a step of reacting the halotetrafluorosulfanyl pyridine with a fluoride source to form pentafluorosulfanyl pyridine.

6. The process according to claim 5, wherein the halogen to be reacted with pyridyldisulfide is chlorine (Cl₂).

7. The process according to claim 5, wherein the fluoride salt represented by formula (b) is alkali metal fluoride.

8. The process according to any one of claims 5 to 7, wherein the fluoride source is hydrogen fluoride.

## Patentansprüche

1. Pentafluorsulfanylpyridin, dargestellt durch Formel (d): 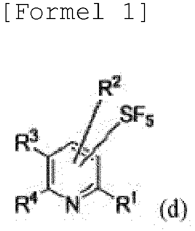
worin eine SF₅-Gruppe an einer von Position 3 oder Position 4 eines Pyridinrings bindet, und R² an der anderen von Position 3 oder Position 4 bindet und
R¹, R², R³ und R⁴ unabhängig ein Wasserstoffatom, ein Halogenatom, eine Alkylgruppe mit 1 bis 18 Kohlenstoffatomen, eine Arylgruppe mit 6 bis 30 Kohlenstoffatomen, eine Nitrogruppe, eine Cyanogruppe, einer Alkansulfonylgruppe mit 1 bis 18 Kohlenstoffatomen, eine Arensulfonylgruppe mit 6 bis 30 Kohlenstoffatomen, eine Alkoxygruppe mit 1 bis 18 Kohlenstoffatomen, eine Aryloxygruppe mit 6 bis 30 Kohlenstoffatomen, eine Acyloxygruppe mit 1 bis 18 Kohlenstoffatomen, eine Alkansulfonyloxygruppe mit 1 bis 18 Kohlenstoffatomen, eine Arensulfonyloxygruppe mit 6 bis 30 Kohlenstoffatomen, eine Alkoxycarbonylgruppe mit 2 bis 18 Kohlenstoffatomen, eine Aryloxycarbonylgruppe mit 7 bis 30 Kohlenstoffatomen, eine Benzylaminogruppe, eine Aminogruppe, eine Azidgruppe, eine Aralkyloxygruppe mit 7 bis 30 Kohlenstoffatomen, eine Aralkylsulfidgruppe mit 7 bis 30 Kohlenstoffatomen oder eine SF₅-Gruppe sind),
worin mindestens eines von R¹ und R⁴ ein Fluoratom ist.

2. Pentafluorsulfanylpyridin gemäß Anspruch 1, das 2-Fluor-3-pentafluorsulfanylpyridin, 2,6-Difluor-3-pentafluorsulfanylpyridin oder 6-Fluor-3-pentafluorsulfanylpyridin ist.

3. Chlortetrafluorsulfanylpyridin, dargestellt durch Formel (c'): 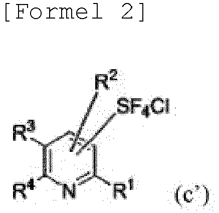
worin eine SF₄Cl-Gruppe an einer von Position 3 oder Position 4 eines Pyridinrings bindet und R² an die andere von Position 3 oder Position 4 bindet und
R¹, R², R³ und R⁴ unabhängig ein Wasserstoffatom, ein Halogenatom, eine Alkylgruppe mit 1 bis 18 Kohlenstoffatomen, eine Arylgruppe mit 6 bis 30 Kohlenstoffatomen, eine Nitrogruppe, eine Cyanogruppe, einer Alkansulfonylgruppe mit 1 bis 18 Kohlenstoffatomen, eine Arensulfonylgruppe mit 6 bis 30 Kohlenstoffatomen, eine Alkoxygruppe mit 1 bis 18 Kohlenstoffatomen, eine Aryloxygruppe mit 6 bis 30 Kohlenstoffatomen, eine Acyloxygruppe mit 1 bis 18 Kohlenstoffatomen, eine Alkansulfonyloxygruppe mit 1 bis 18 Kohlenstoffatomen, eine Arensulfonyloxygruppe mit 6 bis 30 Kohlenstoffatomen, eine Alkoxycarbonylgruppe mit 2 bis 18 Kohlenstoffatomen, eine Aryloxycarbonylgruppe mit 7 bis 30 Kohlenstoffatomen, eine Benzylaminogruppe, eine Aminogruppe, eine Azidgruppe, eine Aralkyloxygruppe mit 7 bis 30 Kohlenstoffatomen, eine Aralkylsulfidgruppe mit 7 bis 30 Kohlenstoffatomen oder eine SF₅-Gruppe sind),
worin mindestens eines von R¹ und R⁴ ein Fluoratom ist.

4. Chlortetrafluorsulfanylpyridin gemäß Anspruch 3, das 2-Fluor-3-chlortetrafluorsulfanylpyridin, 2,6-Difluor-3-chlortetrafluorsulfanylpyridin oder 6-Fluor-3-chlortetrafluorsulfanylpyridin ist.

5. Herstellungsverfahren für Pentafluorsulfanylpyridin gemäß Anspruch 1, umfassend:
einen Schritt zum Reagieren einer durch Formel (a) dargestellten Pyridyldisulfidverbindung: 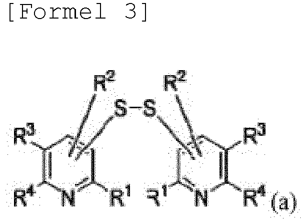
worin R¹, R², R³ und R⁴ wie oben definiert sind,
mit einem Halogen, ausgewählt aus der Gruppe, bestehend aus Chlor, Brom und Iod, oder einer Interhalogenverbindung ausgewählt aus der Gruppe, bestehend aus ClF, BrF, ClBr, ClI, Cl₃I und CrI, und
einem durch Formel (b) dargestellten Fluoridsalz:
M⁺F⁻ (b)
worin M ein Metallatom, eine Ammoniumgruppe oder eine Phosphoniumgruppe ist,
um Halogentetrafluorsulfanylpyridin, dargestellt durch Formel (c), zu bilden: 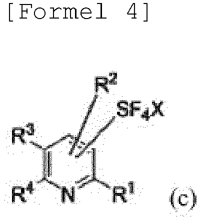
worin R¹, R², R³ und R⁴ wie oben definiert sind und x ein Chloratom, ein Bromatom oder ein Iodatom ist, und
einen Schritt zum Reagieren des Halogentetrafluorsulfanylpyridins mit einer Fluoridquelle, um Pentafluorsulfanylpyridin zu bilden.

6. Verfahren gemäß Anspruch 5, worin das Halogen, das mit Pyridyldisulfid reagieren soll, Chlor (Cl₂) ist.

7. Verfahren gemäß Anspruch 5, worin das durch Formel (b) dargestellte Fluoridsalz ein Alkalimetallfluorid ist.

8. Verfahren gemäß irgendeinem der Ansprüche 5 bis 7, worin die Fluoridquelle Fluorwasserstoff ist.

## Revendications

1. Pentafluorosulfanylpyridine représentée par la formule (d) : 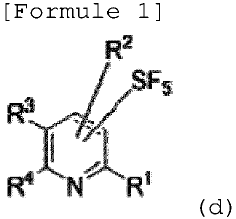
dans laquelle un groupe SF₅ est lié à l'une de la position 3 ou la position 4 d'un cycle pyridine, et R² est lié à l'autre de la position 3 ou la position 4 ; et
R¹, R², R³ et R⁴ sont indépendamment un atome d'hydrogène, un atome d'halogène, un groupe alkyle ayant 1 à 18 atomes de carbone, un groupe aryle ayant 6 à 30 atomes de carbone, un groupe nitro, un groupe cyano, un groupe alcanesulfonyle ayant 1 à 18 atomes de carbone, un groupe arènesulfonyle ayant 6 à 30 atomes de carbone, un groupe alcoxy ayant 1 à 18 atomes de carbone, un groupe aryloxy ayant 6 à 30 atomes de carbone, un groupe acyloxy ayant 1 à 18 atomes de carbone, un groupe alcanesulfonyloxy ayant 1 à 18 atomes de carbone, un groupe arènesulfonyloxy ayant 6 à 30 atomes de carbone, un groupe alcoxycarbonyle ayant 2 à 18 atomes de carbone, un groupe aryloxycarbonyle ayant 7 à 30 atomes de carbone, un groupe benzylamino, un groupe amino, un groupe azide, un groupe aralkyloxy ayant 7 à 30 atomes de carbone, un groupe sulfure d'aralkyle ayant 7 à 30 atomes de carbone, ou un groupe SF₅) ;
dans laquelle au moins l'un de R¹ et R⁴ est un atome de fluor.

2. Pentafluorosulfanylpyridine selon la revendication 1 qui est la 2-fluoro-3-pentafluorosulfanylpyridine, la 2,6-difluoro-3-pentafluorosulfanylpyridine ou la 6-fluoro-3-pentafluorosulfanylpyridine.

3. Chlorotétrafluorosulfanylpyridine représentée par la formule (c') : 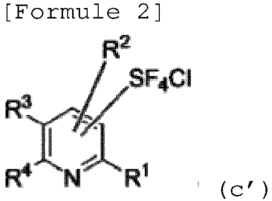
dans laquelle un groupe SF₄Cl est lié à l'une de la position 3 ou la position 4 d'un cycle pyridine, et R² est lié à l'autre de la position 3 ou la position 4 ; et
R¹, R², R³ et R⁴ sont indépendamment un atome d'hydrogène, un atome d'halogène, un groupe alkyle ayant 1 à 18 atomes de carbone, un groupe aryle ayant 6 à 30 atomes de carbone, un groupe nitro, un groupe cyano, un groupe alcanesulfonyle ayant 1 à 18 atomes de carbone, un groupe arènesulfonyle ayant 6 à 30 atomes de carbone, un groupe alcoxy ayant 1 à 18 atomes de carbone, un groupe aryloxy ayant 6 à 30 atomes de carbone, un groupe acyloxy ayant 1 à 18 atomes de carbone, un groupe alcanesulfonyloxy ayant 1 à 18 atomes de carbone, un groupe arènesulfonyloxy ayant 6 à 30 atomes de carbone, un groupe alcoxycarbonyle ayant 2 à 18 atomes de carbone, un groupe aryloxycarbonyle ayant 7 à 30 atomes de carbone, un groupe benzylamino, un groupe amino, un groupe azide, un groupe aralkyloxy ayant 7 à 30 atomes de carbone, un groupe sulfure d'aralkyle ayant 7 à 30 atomes de carbone, ou un groupe SF₅) ;
dans laquelle au moins l'un de R¹ et R⁴ est un atome de fluor.

4. Chlorotétrafluorosulfanylpyridine selon la revendication 3 qui est la 2-fluoro-3-chlorotétrafluorosulfanylpyridine, la 2,6-difluoro-3-chlorotétrafluorosulfanylpyridine, ou la 6-fluoro-3-chlorotétrafluorosulfanylpyridine.

5. Procédé de fabrication de la pentafluorosulfanylpyridine selon la revendication 1 comprenant :
une étape de réaction d'un composé de disulfure de pyridyle représenté par la formule (a) : 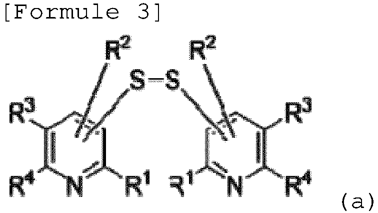
dans laquelle R¹, R², R³ et R⁴ sont tels que définis ci-dessus,
avec un halogène choisi dans un groupe constitué de chlore, brome et iode, ou un composé interhalogène choisi dans un groupe constitué de ClF, BrF, ClBr, ClI, Cl₃I et BrI, et
un sel de fluorure représenté par la formule (b) :
M⁺F⁻ (b)
dans laquelle M est un atome de métal, un groupe ammonium ou un groupe phosphonium
pour former une halogénotétrafluorosulfanylpyridine représentée par la formule (c) : 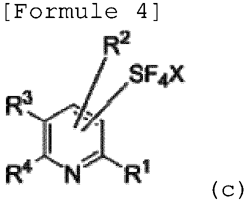
dans laquelle R¹, R², R³ et R⁴ sont tels que définis ci-dessus, et X est un atome de chlore, un atome de brome ou un atome d'iode ; et
une étape de réaction de l'halogénotétrafluorosulfanylpyridine avec une source de fluorure pour former la pentafluorosulfanylpyridine.

6. Procédé selon la revendication 5, dans lequel l'halogène devant réagir avec le disulfure de pyridyle est le chlore (Cl₂).

7. Procédé selon la revendication 5, dans lequel le sel de fluorure représenté par la formule (b) est un fluorure de métal alcalin.

8. Procédé selon l'une quelconque des revendications 5 à 7, dans lequel la source de fluorure est le fluorure d'hydrogène.
